# EUROPEAN PATENT APPLICATION

(11) **EP 1 867 327 A1**
(43) Date of publication of application: **19.12.2007**
(21) Application number: 07011342.8
(22) Date of filing: 09.06.2007
(51) Int. Cl.: A61K 31/122, A61P 25/16

(54) **Astaxanthin and esters thereof for protecting neurocytes and treating Parkinson's disease**

(30) Priority: 16.06.2006 JP 2006166924
(71) Applicant: Yamaha Hatsudoki Kabushiki Kaisha, Iwata-shi, Shizuoka 438-8501 (JP); Tokyo Metropolitan Foundation for Research on Aging and Promotion of Human Welfare, Tokyo 173-0015 (JP)
(72) Inventor: Tsuji, Shinji, Iwata-shi Shizuoka 438-8501 (JP); Shirasawa, Takuji c/o Tokyo Metropolitan Institute of Gerontology, Tokyo Metropolitan Foundation, Tokyo 173-0015 (JP); Shmizu, Takahiko c/o Tokyo Metropolitan Institute of Gerontology, Tokyo Metropolitan Foundation, Tokyo 173-0015 (JP)
(74) Representative: Schlich, George William

(57) **Abstract**

A neurocyte protective agent that is capable of alleviating mitochondrial dysfunction and oxidative stress in neurocytes is provided. The neurocyte protective agent and the agent for protecting neurodegenerative diseases according to the present invention comprise astaxanthin and/or an ester thereof. In particular, the neurocyte protective agent of the present invention is effective in protecting against the degeneration of dopaminergic neurons of the substantia nigra and noradrenergic neurons of the locus ceruleus, and it is expected that ingestion of the neurocyte protective agent will serve as fundamental therapy for Parkinson's disease.

SEQUENCE LISTING

<110>  Yamaha Hatsudoki Kabushiki Kaisha
      Tokyo Metropolitan Foundation for Research on Aging and Promotion
of Human

<120>  Neurocyte Protective Agent

<130>  PY53248JP0EP0

<150>  JP2006-166924
<151>  2006-06-16

<160>  5

<170>  PatentIn version 3.1

<210>  1
<211>  22
<212>  DNA
<213>  Artificial Sequence

<220>
<223>  P1 primer

<400>  1
cgaggggcat ctagtggaga ag                                              22


<210>  2
<211>  23
<212>  DNA
<213>  Artificial Sequence

<220>
<223>  P2 primer

<400>  2
ttagggctca ggtttgtcca gaa                                             23


<210>  3
<211>  17
<212>  DNA
<213>  Artificial Sequence

<220>
<223>  P4 primer

<400>  3
agcttggctg gacgtaa                                                    17

<210>  4
<211>  21
<212>  DNA
<213>  Artificial Sequence

<220>
<223>  Cre primer (Forward)

<400>  4
cactagctcg agacccatga t                                                21

<210>  5
<211>  18
<212>  DNA
<213>  Artificial Sequence

<220>
<223>  Cre primer (Reverse)

<400>  5
cgccctcttt aaaggcca                                                    18

## Description

### BACKGROUND OF THE INVENTION

### 1. Field of the Invention

The present invention relates to novel neurocyte protective agents that can alleviate mitochondrial dysfunction and oxidative stress in neurons.

### 2. Description of the Related Art

Neurodegenerative diseases are progressive diseases in which specific neurocytes are degenerated and lost. Although the cause of degeneration differs depending on the disease, a common pathological finding that has been reported is the accumulation of abnormal proteins. Examples of such diseases include Alzheimer's disease, polyglutamic disease, Parkinson's disease, and amyotrophic lateral sclerosis.

Parkinson's disease is a neurodegenerative disease the main manifestation of which is a movement disorder that includes four major symptoms: tremors, rigidity, akinesia, and postural instability. Disease onset is usually in the late fifties to sixties, but it has been known to appear from 20 to 80 years of age.

The primary cause of Parkinson's disease is believed to be a dopamine-acetylcholine imbalance caused by the degeneration and loss of dopaminergic neurons in the midbrain substantia nigra, which results in insufficient dopamine-dependant neurotransmission from the midbrain substantia nigra to the corpus striatum in the basal ganglia. Consequently, this neurotransmission is dominated by acetylcholine-dependant neurotransmission. The mechanism of neurodegeneration in the substantia nigra, which is the responsible lesion, has not been elucidated. The prevailing theory on the mechanism of the neurodegeneration is that mitochondrial dysfunction and the accompanying oxidative stress occur within dopamine cells and thereby induce neural cell death in a vicious cycle (P.M. Abou-Sleiman et al. (2006) Nature Reviews Neuroscience Vol. 7, No. 3, pp.207-219). In other words, mitochondrial dysfunction and oxidative stress are regarded as the central mechanism of neurodegeneration.

It is also clear that various impairments other than the above movement disorders may appear as symptoms of Parkinson's disease. Examples include autonomic symptoms such as orthostatic hypotension and pollakiuria; olfactory hypesthesia; sleep disorders such as insomnia, REM sleep behavioral abnormality, and restless legs syndrome; depression; and intellectual dysfunction. Corresponding to these impairments, pathological lesions extend to the peripheral autonomic nerves, the dorsal motor nucleus of the vagus nerve, the olfactory bulb, the locus ceruleus, the raphe nuclei, the Meynert basal nucleus, the amygdala, and even the cerebral cortex.

The morbidity of Parkinson's disease is approximately 100 per 100,000 persons, and Parkinson's disease is the second most common neurodegenerative disease behind Alzheimer's disease. Thus, there is a need for the development of fundamental treatment methods. At the current time, however, there are no pharmaceuticals that cure the disease or stop its progress. The current treatments for Parkinson's disease are not fundamental treatments that interfere with the degeneration of dopamine cells, but rather treat symptoms by correcting insufficiencies in dopamine production. Although other treatments that involve food, exercise, lifestyle, rehabilitation, and surgery exist, pharmacotherapy is the primary treatment.

Pharmacotherapy for Parkinson's disease can take the following approaches: (1) to supply dopamine because its production has been lowered in the corpus striatum; (2) to activate dopamine transmission; and (3) to depress the function of acetylcholine to substantially normal levels to bring the dopamine-acetylcholine disequilibrium close to equilibrium.

Of these approaches, the most effective and common one is (1), and typically consists of levodopa therapy. Levodopa (L-dopa) is a dopamine precursor that is metabolized into dopamine within the brain. Examples of pharmaceuticals that fall into the category (2) are dopamine agonists that act directly on dopamine receptors, MAO-B inhibitors that inhibit monoamine oxidase (MAO-B), which metabolizes dopamine, and dopamine release stimulators. Examples of drugs that fall into the category (3) are cholinergic-blocking agents. Apart from these pharmaceuticals there is also droxidopa, which compensates for the loss of noradrenergic neurons in the locus ceruleus of the pons in addition to the dopamine cells in the midbrain substantia nigra in advanced Parkinson's disease. Droxidopa is a noradrenaline precursor that has an effect on the movement disorder known as frozen gait, and the postural instability known as pulsion, and these impairments are observed in Parkinson's patients with moderate to advanced Parkinson's disease.

Levodopa is highly effective for Parkinson's disease but it has various side effects, and this is one problem with levodopa therapy. Side effects from the administration of levodopa are characterized by the following phenomena (i) and (ii).

### (i) Wearing Off Phenomenon

This refers to the phenomenon that when levodopa is administered over the long term, its period of effectiveness shortens and symptoms suddenly reappear two to three hours after taking levodopa. The reason for this is believed to be that dopamine cell degeneration causes further reductions in cell number, and the levodopa in the brain is metabolized in a short time by other cells without being retained in dopamine cells. In other words, in a short time the levodopa leads to a large amount of dopamine and acts on dopamine receptors, so that involuntary movement (dyskinesia) appears due to dopamine overactivity. This is followed by akinesis due to the sudden reduction in dopamine. Simply increasing the amount of levodopa often causes symptoms to worsen.

### (ii) On-Off Phenomenon

This refers to the phenomenon of repeated sudden alleviation and deterioration of symptoms regardless of the administration period or the blood concentration. It is believed that this is caused by a change in levodopa absorption and metabolism or a change in the sensitivity of dopamine receptors, but the detailed mechanism of pathogenesis is unclear.

Thus, there are some problems in levodopa therapy, which is currently the most common pharmacotherapy for diseases associated with degeneration of dopamine cells. There is an urgent need for the development of pharmaceuticals that can be applied in fundamental treatments for suppressing the degeneration of dopamine cells themselves.

It has been reported that a depression in motor ability in mice when reperfusing after cerebral ischemia is suppressed by administration of astaxanthin which is known to be an antioxidant (G. Hussein et al. (2005) Biol. Pharm. Bull. Vol. 28, No. 1, pp.47-52). In G. Hussein et al., this result is interpreted as an indication of the antioxidative effect of astaxanthin on the free radicals that are produced by cerebral ischemia, and therefore it is deduced that astaxanthin is effective in protecting neurons. However, G. Hussein et al. did not evaluate which neurocytes degenerated due to cerebral ischemia, or whether the neurocytes were protected. Moreover, since neurodegenerative diseases such as Parkinson's disease are not caused by cerebral ischemia, the effectiveness of astaxanthin with respect to such diseases is unclear.

It has become clear that astaxanthin suppresses lipid peroxide in the blood. Further, astaxanthin has been suggested to be effective against diseases associated with blood lipid peroxide (Japanese Laid-Open Patent Publication No. 2006-8719). Japanese Laid-Open Patent Publication No. 2006-8719 describes Alzheimer's disease and Parkinson's disease along with various cardiovascular diseases as examples of diseases associated with blood lipid peroxide. However, considering that lipid peroxide in the blood is not known to have a direct effect on neurocytes in the brain, and that it is unclear whether lipid peroxides can pass through the blood-brain barrier, it is uncertain from the description of Japanese Laid-Open Patent Publication No. 2006-8719 whether astaxanthin, which has the effect of suppressing lipid peroxide in the blood, is actually effective against neurodegenerative diseases such as Parkinson's disease.

Similarly, it has been suggested that carotenoid analogs prevent the occurrence of and/or alleviate diseases related to the production of reactive oxygen species, reactive nitrogen species, and radicals and/or non-radicals (WO 2004/011423). In WO 2004/011423, various diseases are listed as examples of diseases that can be alleviated by carotenoid analogs. However, the degeneration of neurocytes in the brain is not examined. Thus, similarly to the disclosure in Japanese Laid-Open Patent Publication No. 2006-8719, it is uncertain whether these carotenoid analogs are actually effective against neurodegenerative diseases such as Parkinson's disease.

### SUMMARY OF THE INVENTION

It is an object of the present invention to provide a neurocyte protective agent that can alleviate mitochondrial dysfunction and oxidative stress in neurocytes.

The present invention provides a neurocyte protective agent for a neurocyte comprising astaxanthin and/or an ester thereof.

The present invention also provides a method for protecting a neurocyte, comprising administering an effective amount of astaxanthin and/or an ester thereof to an individual.

The present invention further provides a use of astaxanthin and/or an ester thereof in the manufacture of a neurocyte protective agent for a neurocyte.

In one embodiment, the neurocyte is a dopaminergic neuron of the substantia nigra or a noradrenergic neuron of the locus ceruleus.

The present invention further provides an agent for preventing a neurodegenerative disease comprising astaxanthin and/or an ester thereof.

The present invention also provides a method for preventing a neurodegenerative disease, comprising administering a prophylactically effective amount of astaxanthin and/or an ester thereof to an individual.

The present invention further provides a use of astaxanthin and/or an ester thereof in the manufacture of an agent for preventing a neurodegenerative disease.

In one embodiment, the neurodegenerative disease is Parkinson's disease.

According to the present invention, a novel neurocyte protective agent that is capable of alleviating mitochondrial dysfunction and oxidative stress in neurocytes is provided. Mitochondrial dysfunction and oxidative stress are thought to be the principal mechanism through which neurodegeneration occurs in Parkinson's disease. Thus, it can be expected that the neurocyte protective agent of the present invention can be used in fundamental therapy for Parkinson's disease. Therefore, the neurocyte protective agent can be used as an agent for preventing neurodegenerative diseases. The neurocyte protective agent of the present invention has very low toxicity, and thus has very high degree of safety and can be consumed in food for a long time.

### BRIEF DESCRIPTION OF THE DRAWINGS

Fig. 1 shows electrophoretic photographs displaying the results of genotyping used to distinguish between a control mouse (F/F mouse) and a TH positive cell specific MnSOD-deficient mouse (KO mouse).
Fig. 2 shows microphotographs of frozen sections of brain including the locus ceruleus from a control mouse (F/F mouse) and a TH positive cell specific MnSOD-deficient mouse (KO mouse) after immunohistochemical staining with MnSOD antibody.
Fig. 3 is a graph showing the change in body weight for the KO mice of the YAX group and the control group.
Fig. 4 is a graph showing the results of a life span analysis for the KO mice of the YAX group and the control group.
Fig. 5 is a graph showing the results of a footprinting test for one each of a KO mouse of the YAX group, a KO mouse of the control group, and an F/F mouse (control mouse).

### DESCRIPTION OF THE PREFERRED EMBODIMENTS

Astaxanthin and/or an ester thereof contained in the neurocyte protective agent of the present invention is a carotenoid represented by the following formula: 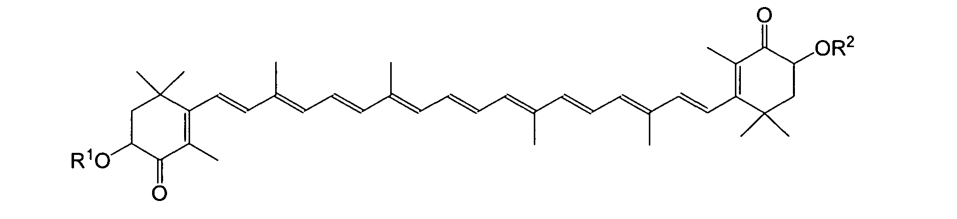 wherein R¹ and R² are both hydrogen in the case of astaxanthin, and R¹ and R² are each independently a hydrogen atom or a fatty acid residue provided that at least one of R¹ and R² is a fatty acid residue in the case of an ester of astaxanthin. Examples of the fatty acid residue in the ester of astaxanthin include, but are not limited to, saturated fatty acids such as palmitic acid and stearic acid or unsaturated fatty acids such as oleic acid, linoleic acid, α-linolenic acid, γ-linolenic acid, bishomo-γ-linolenic acid, arachidonic acid, eicosapentaenoic acid, and docosahexaenoic acid. The astaxanthin ester used in the present invention can be any mono or diester, homogeneous or nonhomogeneous. Astaxanthin has a structure in which an additional oxo group and an additional hydroxy group are present at each end of a β-carotene molecule, so that unlike for β-carotene, the stability of the molecule is low. On the other hand, an ester form (e.g., as obtained in an extract from krill) in which the hydroxy groups at both ends are esterified with an unsaturated fatty acid is more stable.

Astaxanthin and/or an ester thereof used in the present invention may be chemically synthesized or derived from a naturally-occurring product. Examples of the naturally-occurring products in the latter case include red yeast; the shell of crustaceans such as Tigriopus (red water flea) and krills; and microalgae such as green algae, which contain astaxanthin and/or an ester thereof. In the present invention, as long as the properties of astaxanthin and/or esters thereof can be utilized, any extract containing astaxanthin and/or esters thereof produced by any method can be used. Generally, extracts from those naturally-occurring products can be used, and the extracts may be crude or purified if necessary. In the present invention, a crude extract or a crushed powder of naturally-occurring products, or a purified product or a chemically synthesized product, if necessary, that contains such astaxanthin and/or esters thereof can be used either alone or in combination. In view of the chemical stability, an ester form of astaxanthin is preferably used.

The neurocyte protective agent of the present invention can protect neurocytes from progressive degeneration. Examples of neurocytes include cells that are present in the midbrain substantia nigra, the dorsal motor nucleus of the vagus nerve, the olfactory bulb, the locus ceruleus, the raphe nuclei, the Meynert basal nucleus, the amygdala, the cerebral cortex, and periphery autonomic nerves. In particular, it is preferably applied to dopaminergic neurons of the substantia nigra and noradrenergic neurons of the locus ceruleus.

The neurocyte protective agent of the present invention is useful for preventing diseases or symptoms involved in neurodegeneration, and may be used as an agent for preventing a neurodegenerative disease. Examples of such neurodegenerative diseases include Parkinson's disease, Alzheimer's disease, and amyotrophic lateral sclerosis. In particular, the neurocyte protective agent of the present invention may be preferably used against Parkinson's disease.

The route of administration of the neurocyte protective agent or the agent for preventing a neurodegenerative disease according to the present invention may be either oral or parenteral. The dosage form is selected appropriately according to the route of administration. Examples thereof include parenteral solutions, infusion solutions, powders, granules, tablets, capsules, pills, enteric-coated preparations, troches, liquids for internal use, suspensions, emulsions, syrups, liquids for external use, poultices, nose drops, ear drops, eye drops, inhalants, ointments, lotions, suppositories, and enteral nutrients. These can be used either alone or in combination depending on the condition of a disease. To prepare these dosage forms, auxiliary substances commonly used in the field of pharmaceutical manufacturing technology, such as excipients, binders, antiseptics, antioxidants, disintegrators, lubricants, and flavoring agents, can be used as necessary.

The dose of the neurocyte protective agent or the agent for preventing a neurodegenerative disease according to the present invention varies depending on the purpose of administration or the individual to be administered (sex, age, body weight, etc.). The agent is administered in an amount effective for protecting neurocytes or for preventing neurodegenerative diseases. Usually, the dose for an adult in terms of free or unesterified form of astaxanthin may be 0.1 mg to 2 g, preferably 4 mg to 500 mg per day in the case of oral administration, while it may be 0.01 mg to 1 g, preferably 0.1 mg to 500 mg per day in the case of parenteral administration.

The neurocyte protective agent or the agent for preventing a neurodegenerative disease according to the present invention can be used not only as pharmaceuticals as described above, but also as the category of products regulated as "quasi-drugs", cosmetics, functional food products, nutritional supplements, foods and drinks, and other similar products. When used as quasi-drugs or cosmetics, the agent may be used in conjunction with various auxiliary substances commonly used in the field of quasi-drugs or cosmetics, or other technologies, if necessary. Alternatively, when used as functional food products, nutritional supplements, or foods and drinks, the agent may be used in conjunction with additives commonly used for food products, for example, sweeteners, spices, seasonings, antiseptics, preservatives, germicides, and antioxidants, if necessary. The agent may be used in a desired form such as solution, suspension, syrup, granule, cream, paste, or jelly, or may be shaped, if necessary. The ratio of the agent contained in these products is not particularly limited, and can be selected appropriately according to the intended purpose, the mode of usage, and the amount of usage.

### Examples

### Preparation Example 1: Generation of MnSOD-deficient mice

Tyrosine hydroxylase (hereinafter referred to as TH) is an enzyme that acts in the first step of the catecholamine biosynthesis system in which levodopa (L-dopa) is produced from L-tyrosine. TH is subject to feedback inhibition by its end products (i.e., dopamine, noradrenaline, and adrenaline). Thus, TH is the rate limiting enzyme in this biosynthesis system. TH is essential in cells that contain catecholamines, that is, dopamine, noradrenaline, and adrenaline. Cells that contain TH are called tyrosine hydroxylase positive cells, and in the central nervous system the corresponding cells are dopamine cells and noradrenaline cells.

Reactive oxygen species (ROS) that are produced in vivo are primarily leaked from mitochondria in cells, and manganese superoxide dismutase (hereinafter referred to as MnSOD) is the enzyme that converts superoxide (O₂·), one of the ROS, into H₂O₂ within mitochondria. Thus, MnSOD-deficient mice, in which it is thought that MnSOD has been specifically deficient in dopamine cells of the substantia nigra and noradrenaline cells of the locus ceruleus, will be directly attacked by superoxide. Therefore, it can be expected that dysfunction of mitochondria is induced so that the function of neurocytes is disrupted and cell death (apoptosis or necrosis) occurs.

As genetically modified mice serving as models for neurodegeneration, in which MnSOD has been specifically deficient in dopamine cells of the substantia nigra and noradrenaline cells of the locus ceruleus in the central nervous system, tyrosine hydroxylase (TH) positive cell specific MnSOD-deficient mice were generated based on the method described in WO 2004/014131. Specifically, first, a homozygous MnSOD flox mouse (lox/lox, +/+) was mated with a TH-Cre transgenic mouse controlled by a TH (tyrosine hydroxylase) promoter, so that heterozygous mice (lox/w, Cre/+) having the TH-Cre transgene were generated. Then, the heterozygous mouse and a MnSOD flox mouse were mated to generate the TH positive cell specific MnSOD-deficient mice (lox/lox, Cre/+) of interest.

Genotyping was performed to distinguish between the TH positive cell specific MnSOD-deficient mice (lox/lox, Cre/+) (hereinafter referred to as KO mice) and the control mice (MnSOD flox mice (lox/lox, +/+); hereinafter referred to as F/F mice). Specifically, PCR was performed on a digested mouse tail of each mouse immediately after birth by using a primer set (SEQ ID NOs: 1 and 2 or SEQ ID NOs: 1 and 3) that recognizes the MnSOD flox allele (lox) and a primer set (SEQ ID NOs: 4 and 5) that recognizes the TH-Cre allele (Cre), and then the amplified gene fragments corresponding to these genotypes were confirmed by electrophoresis.

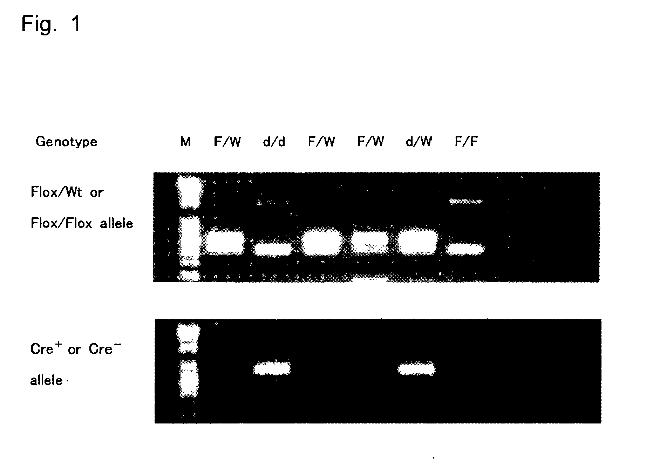

Photographs of the genotyping electrophoresis are shown in Fig. 1. The upper photograph in Fig. 1 displays the result when a lox allele detection primer was used, and the lower photograph displays the result when a Cre allele detection primer was used. The M in Fig. 1 shows the molecular weight markers. From these results, it was confirmed that, as shown in the upper part of Fig. 1, F/F is DNA derived from a mouse homozygous for the MnSOD flox allele, that is, an MnSOD flox mouse (lox/lox, +/+) (F/F mouse), F/W is DNA derived from a mouse heterozygous for the MnSOD flox allele, d/W is DNA derived from a mouse heterozygous for the MnSOD-deficient allele, and d/d is DNA derived from a mouse homozygous for the MnSOD-deficient allele, that is, a MnSOD-deficient mouse (KO mouse).

It was observed that the TH positive cell specific MnSOD-deficient mice (KO mice) thus generated clearly showed phenotypic abnormalities in indices such as life span, body weight, and behavior (refer to the control group in Example 1 below).

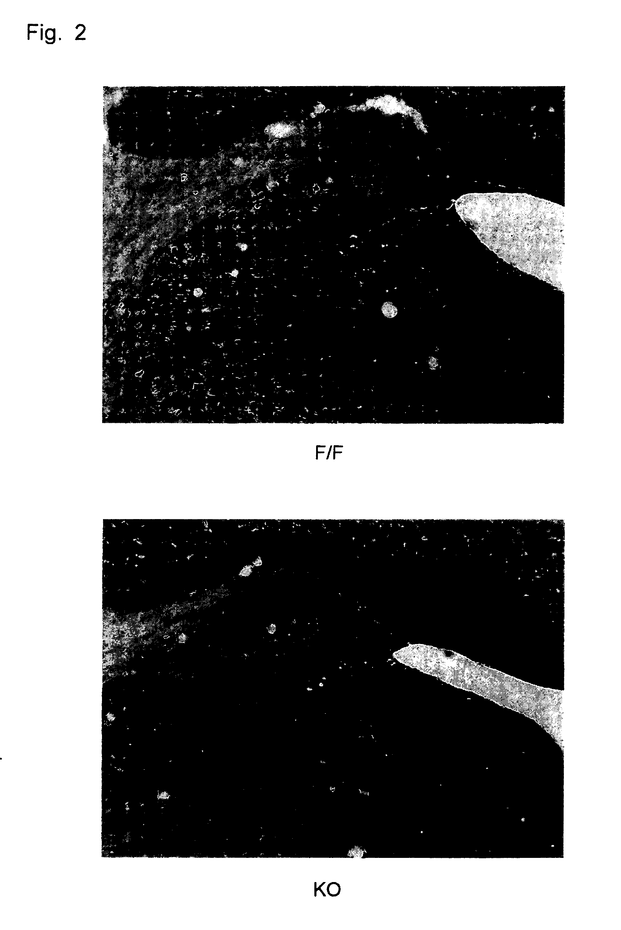

Next, to confirm tissue specific MnSOD deficiency, the brain was removed from four-week old TH positive cell specific MnSOD-deficient mice (KO mice) and control mice (MnSOD flox mice (lox/lox, +/+); F/F mice), frozen and sectioned, and immunohistochemical staining was performed with TH antibody and MnSOD antibody. The results of immunohistochemical staining with MnSOD antibody are shown in Fig. 2.

In the KO mice, the reactivity to the TH antibody was observed in both the substantia nigra and the locus ceruleus, and it failed to confirm the death of TH positive cells. However, it is clear from comparison of the portions indicated by the arrows in Fig. 2 that with anti-MnSOD staining, the stainability in noradrenaline cells of the locus ceruleus was markedly lowered. Also, although not as much as in the locus ceruleus, it was observed that the stainability was lowered in the dopamine cells of the substantia nigra as well. To analyze more closely the deficiency of MnSOD and cell death in the locus ceruleus and the substantia nigra, fluorescent double immunostaining was performed with TH antibody and MnSOD antibody. The sites stained by TH antibody and the sites stained by MnSOD antibody overlapped in the F/F mice, whereas in the KO mice it was clearly confirmed that the expression of TH and the expression of MnSOD do not match in the locus ceruleus. This demonstrated that in the KO mice, MnSOD clearly has been deficient in the noradrenaline cells of the locus ceruleus. The loss of noradrenaline cells themselves also was confirmed in the KO mice. In other words, this indicates that there is a possibility that the mitochondria of the noradrenaline cells have been damaged by reactive oxygen species. The same tendency was confirmed in the substantia nigra as well. It is clear from the above phenomena that the function of the noradrenaline cells of the locus ceruleus and the dopamine cells of the substantia nigra has been impaired.

Parkinson's disease is thought to occur due to mitochondrial dysfunction in dopamine cells and noradrenaline cells, and due to the accompanying oxidative stress. Therefore, the KO mice can serve as useful animal models for elucidating Parkinson's disease and for screening therapeutic agent for Parkinson's disease.

### Preparation Example 2: Preparation of Astaxanthin

Astaxanthin was prepared in the following manner. *Haematococcus pluvialis* K0084 strain was cultivated at 25°C under irradiation with light while bubbling a gas containing 3 vol% CO₂ into the medium and under nutrient stress condition (i.e. nitrogen source deprivation), and then was encysted. The encysted cells were disrupted by means commonly used by those skilled in the art, and a lipophilic fraction containing astaxanthin was extracted with ethanol. The extract was concentrated under reduced pressure, and the ethanol was evaporated to give an extract containing primarily triglyceride in which astaxanthin was contained in an amount of 8% by weight expressed in terms of free form. This extract containing astaxanthin hereinafter will be called YAX (Yamaha AstaXanthin).

### Example 1: Examination of the Effect of Administering Astaxanthin to KO Mice

The KO mice generated in Preparation Example 1 were given astaxanthin, and the effect of astaxanthin on body weight changes, life span, and behavior was examined.

The YAX prepared in Preparation Example 2 was used as the astaxanthin, and this was administered with the diet. That is, YAX was added to powder diet (CRF-1 powder; Oriental Yeast Co., Ltd.) to a concentration of 10 wt% and kneaded until uniform, and the mice were allowed to feed freely on the thus obtained diet from the period immediately after weaning (four weeks old). Expressed in terms of body weight, the amount administered was approximately 1,500 mg/kg/day. KO mice in the control group were given powder diet not supplemented with YAX. It should be noted that for comparison, the KO mice in the control group fed with an ordinary diet not containing YAX were monitored in the same manner.

The body weight was measured once a week, and a Student's t-test was performed. The life span was analyzed using the Kaplan-Meier method. The Log-rank test was employed as the test of the Kaplan-Meier method. The behavior was analyzed by the footprinting test. The footprinting test is a technique for evaluating gait disorders due to dyskinesia. Specifically, ink is applied to the hind feet of the mice and the mice are allowed to walk, and the width of the footprints left behind is analyzed. Student's t-test was employed for the footprinting test.

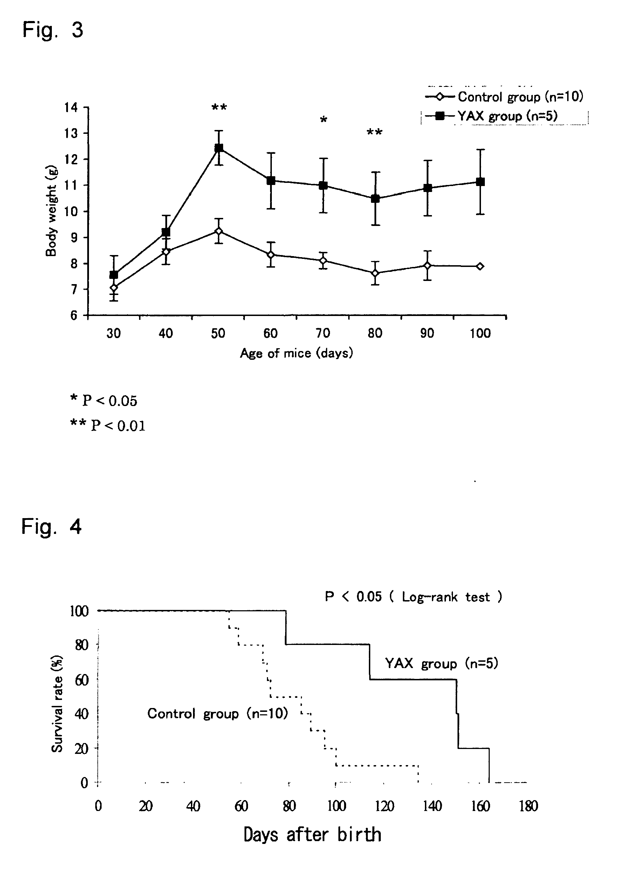

Fig. 3 shows the change in mean body weight of 5 mice of the YAX group and 10 mice of the control group, respectively. In mice of the control group, which did not ingest YAX, the increase in body weight during the growth period was slow, and the decrease in body weight was observed from about day 50 after birth. On the contrary, body weight in the YAX group increased significantly when compared to the control group at 50 days, 70 days, and 80 days (p<0.05), and decrease in body weight was suppressed. In particular, at day 50, their body weight was increased significantly at p<0.01, and it was clear that low body weight due to growth abnormalities had been suppressed by intake of YAX.

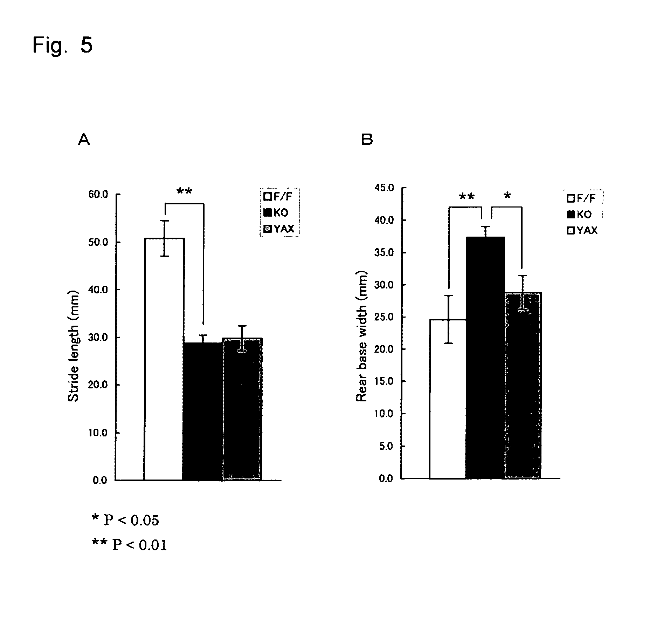

Fig. 4 shows the results of an analysis of the life span of 5 mice of the YAX group and 10 mice of the control group, respectively. In the control group, the life span was short and the survival rate gradually reduced. In the YAX group, the life span was significantly extended (p<0.05) and the survival rate remained high. It can be seen that the administration of astaxanthin according to the invention not only extends the life span but also increases the survival rate.

Fig. 5 shows the results of the footprinting test in one each of an approximately 16-week old (approximately 110 days) KO mouse of the YAX group, a KO mouse of the control group, and an F/F mouse (control mouse). It should be noted that the control mouse was an F/F mouse given an ordinary diet. Fig. 5 shows the mean values, calculated from five-point measurements, for the stride length (step width) (A), and for the rear base width (left/right width) (B). The KO mouse (control group) clearly had a smaller stride length, that is, step width when walking, than the control F/F mouse. On the other hand, the rear base width of the KO mouse was larger, and this is likely because the width between the left and right legs became large due to difficulty of supporting its body with its legs. The YAX-administered KO mouse (YAX group) had the same stride length as the control group, but its rear base width was smaller than the control group. Thus, it can be appreciated that by administering YAX, movement abnormalities have been reduced in the mice that can serve as Parkinson's disease models.

The above discussion demonstrates that astaxanthin has powerful neurocyte protective activity in TH positive cell specific MnSOD-deficient mice, particularly with respect to damage from reactive oxygen species, which is regarded as the main cause of Parkinson's disease. It was thus appreciated that a neurocyte protective effect can be expected with just the ingestion of astaxanthin.

According to the present invention, a novel neurocyte protective agent that is capable of alleviating mitochondrial dysfunction and oxidative stress in neurocytes is provided. This neurocyte protective agent can be used as an agent for preventing neurodegenerative diseases. In particular, mitochondrial dysfunction and oxidative stress are thought to be the principal mechanism through which neurodegeneration occurs in Parkinson's disease. Thus, it can be expected that the neurocyte protective agent or the agent for preventing neurodegenerative diseases according to the present invention can be used in fundamental therapy for Parkinson's disease. The astaxanthin and/or ester thereof that serves as the neurocyte protective agent or the agent for preventing neurodegenerative diseases according to the present invention has been consumed in food for a long time and has very low toxicity, and thus has high degree of safety. Accordingly, these agents can be used not only as pharmaceuticals but also prophylactically on a daily basis as health food products.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. A neurocyte protective agent for a neurocyte comprising astaxanthin and/or an ester thereof.

2. The neurocyte protective agent of claim 1, wherein the neurocyte is a dopaminergic neuron of the substantia nigra or a noradrenergic neuron of the locus ceruleus.

3. An agent for preventing a neurodegenerative disease comprising astaxanthin and/or an ester thereof.

4. The agent of claim 3, wherein the neurodegenerative disease is Parkinson's disease.

5. Use of astaxanthin and/or an ester thereof in the manufacture of a neurocyte protective agent for a neurocyte.

6. The use of claim 5, wherein the neurocyte is a dopaminergic neuron of the substantia nigra or a noradrenergic neuron of the locus ceruleus.

7. Use of astaxanthin and/or an ester thereof in the manufacture of an agent for preventing a neurodegenerative disease.

8. The use of claim 7, wherein the neurodegenerative disease is Parkinson's disease.
